(19) **Europäisches Patentamt** / **European Patent Office** / **Office européen des brevets**

(11) **EP 4 647 004 A1**

(12) **DEMANDE DE BREVET EUROPEEN**

(43) Date de publication:
**12.11.2025  Bulletin 2025/46**

(21) Numéro de dépôt: **25174281.3**

(22) Date de dépôt: **05.05.2025**

(51) Classification Internationale des Brevets (IPC):
**A61B 5/0536** (2021.01)    **G06N 3/0464** (2023.01)

(52) Classification Coopérative des Brevets (CPC):
**A61B 5/0536; G06N 3/0455; G06N 3/0464**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **06.05.2024  FR 2404751**

(71) Demandeurs:
- **Commissariat à l'Energie Atomique et aux Energies Alternatives**
  **75015 Paris (FR)**
- **Centre National de la Recherche Scientifique**
  **75016 Paris (FR)**

- **Université d'Aix Marseille**
  **13007 Marseille (FR)**
- **Centrale Méditerranée**
  **13013 Marseille (FR)**

(72) Inventeur: **SAOUDI, Anès**
**13115 SAINT PAUL LEZ DURANCE (FR)**

(74) Mandataire: **Ipsilon**
**Le Centralis**
**63, avenue du Général Leclerc**
**92340 Bourg-la-Reine (FR)**

(54) **PROCÉDÉ DE RECONSTRUCTION DE LA DISTRIBUTION DES PROPRIÉTÉS ÉLECTRIQUES DES MATÉRIAUX UTILISANT LA TOMOGRAPHIE PAR IMPÉDANCE ÉLECTRIQUE**

(57) L'invention porte sur un procédé de reconstruction de la distribution des propriétés électriques d'au moins un matériau d'un corps comportant une partie cylindrique contenant un fluide, utilisant un réseau de neurones et des données de valeurs électriques du corps mesurées au préalable par tomographie par impédance électrique à l'aide d'électrodes agencées autour d'une périphérie de la partie cylindrique du corps, chaque électrode ayant été excitée par un potentiel de forme prédéfinie,

le réseau de neurones comportant :
- un auto-encodeur comprenant plusieurs niveaux $(L_1,...,L_J)$ comportant chacun au moins une couche de convolution et au moins une couche d'abandon, et
- un module d'attention comprenant des portes d'attention $(AG_1,..., AG_{J-1})$, ayant chacun un signal d'attention $(g_j)$ associé.

[Fig 2]

Fig. 2

## Description

### Domaine technique

La présente invention concerne le domaine de la tomographie par impédance électrique.

L'invention porte plus particulièrement sur un procédé de reconstruction de la distribution des propriétés électriques des matériaux d'un corps comportant une partie cylindrique contenant un fluide, utilisant un réseau de neurones et des données de propriétés électriques du corps mesurées au préalable par tomographie par impédance électrique.

L'invention concerne également un produit programme d'ordinateur configuré pour mettre en œuvre ce procédé.

L'application principale visée par la présente invention concerne la surveillance d'écoulements de fluides susceptibles de varier de façon abrupte, comme cela peut être le cas pour des fluides circulant sous haute pression et à haute température.

Une application d'intérêt particulier concerne la surveillance de conduits d'installations nucléaires, mais d'autres applications peuvent être envisagées dans le cadre de l'invention.

### Technique antérieure

La Tomographie par Impédance Electrique (TIE) est une technique non invasive, non destructive, qui permet de visualiser, en temps réel et en continu, l'intérieur d'un objet en mesurant les propriétés électriques (potentiel et courant électrique) de l'objet à sa surface. Cette approche, robuste, est particulièrement adaptée à la réalisation de mesures non intrusives dans des environnements sous hautes pressions et/ou hautes températures.

Par rapport à l'IRM et au scanner, la TIE offre une fréquence d'images rapide de plusieurs kHz, avec une résolution spatiale plus faible en raison de la non-linéarité du problème inverse.

La TIE consiste plus précisément à injecter des courants ou potentiels électriques au moyen d'un ensemble d'électrodes non-intrusives disposées à la surface de l'objet surveillé et ensuite à mesurer les potentiels ou courants électriques à la surface de l'objet.

Les électrodes peuvent être uniquement en contact avec la surface extérieure de l'objet. Toutefois, dans le cas où la surface de l'objet est en métal, les électrodes doivent traverser la paroi et être en contact avec le fluide.

Le modèle d'excitation appliqué à travers les paires d'électrodes, avec les mesures de tension, résulte en une cartographie entre le courant et la tension. La carte de l'impédance à l'intérieur de l'objet est reconstruite en résolvant le problème inverse associé, afin de retrouver la distribution interne de la conductivité et de la permittivité, conformément à la loi d'Ohm.

Initialement, les algorithmes utilisés pour résoudre le problème inverse non linéaire de la TIE étaient principalement des théories mathématiques itératives, telles que la régularisation de Tikhonov (TR), la rétroprojection linéaire (LBP), la méthode de Gauss-Newton (GNM), la méthode d'erreur en une étape de Newton (NOSER). Ces méthodes utilisent l'approximation linéaire de premier ordre pour conjuguer la cartographie non linéaire, ce qui conduit à une faible résolution et à une grande sensibilité au bruit pendant les mesures, sans compter que le temps de calcul exponentiel augmente avec les données.

Au cours des trente dernières années, les méthodes utilisant les réseaux neuronaux artificiels ont permis des avancées majeures dans le domaine de la TIE, en raison de leur capacité à cartographier des relations non linéaires complexes avec une bonne convergence et de faibles erreurs. Cela se traduit par une résolution spatiale plus élevée et un temps d'exécution plus court, non seulement pour le problème inverse, mais aussi pour le débruitage, la super-résolution et la segmentation des images.

La demande FR 3 121 234 décrit une méthode de reconstruction d'image utilisant l'algorithme NOSER et mettant en œuvre un multiplexage en fréquence dans lequel les signaux d'excitation sont imposés simultanément à toutes les électrodes. Pour permettre la discrimination des signaux, chaque électrode est excitée par un signal de forme trigonométrique. Le fait d'exciter toutes les électrodes simultanément évite la redondance que l'on retrouve dans les données obtenues en excitant séquentiellement les électrodes appairées. Cependant, la résolution spatiale obtenue n'est parfois pas suffisante pour détecter certains objets.

L'article [1] propose le réseau ADALINE, basé sur un élément linéaire adaptatif. Les travaux décrits dans l'article [2] ont appliqué les réseaux de rétropropagation, et ceux de l'article [3] ont étudié les perceptrons multicouches bayésiens. Ces études ont été lancées sur des opérateurs de reconstruction de formation linéaire, en mesurant la différence entre les mesures de tension et en évitant la non-linéarité entre la tension et la conductivité.

Pour surmonter la non-linéarité de la TIE, la méthode de l'article [4] utilise le réseau neuronal Radial-Basis-Functions, et celle de l'article [5] a exploré un réseau neuronal dense avec des données simulées d'EIDORS. Les études récentes sont davantage axées sur les architectures complexes, comme décrit dans l'article [6], où est appliquée une méthode d'auto-encodeur finement ajusté à la surveillance pulmonaire EIT, ou dans l'article [7] qui propose un auto-encodeur multicouche. Les chercheurs de l'article [8] ont étudié l'utilisation d'antécédents basés sur l'énergie avec un réseau neuronal informé par la physique pour améliorer l'apprentissage du modèle, tandis que ceux de l'article [9] ont utilisé l'apprentissage hybride-fusion pour la reconstruction à haute résolution.

La demande WO 2022/77866 décrit une méthode d'imagerie par impédance électrique utilisant un ensemble original de données de tension mesurées sur une zone à tester. Une séquence de distribution de conductivité initiale de la zone forme un ensemble de données d'entraînement correspondant, auquel du bruit est ajouté. Un auto-encodeur variationnel est entraîné avec ces données d'entraînement. En fonction de l'ensemble de données de tension d'entrée, un encodeur de l'auto-encodeur variationnel est entraîné pour obtenir une caractéristique de données de tension d'entrée, et une relation de mise en correspondance est établie entre l'ensemble de données de tension d'entrée et une séquence de distribution de conductivité correspondante en utilisant un décodeur de l'auto-encodeur variationnel entraîné.

Avec les progrès continus de l'apprentissage profond dans la TIE, de meilleurs résultats sont explorés par rapport aux algorithmes traditionnels.

De façon connue, le problème inverse est résolu sans tenir compte de l'intérêt de donner des indications à l'intelligence artificielle (IA) sur les points à privilégier pour améliorer la résolution spatiale du résultat.

L'amélioration de la reconstruction de l'image est pourtant primordiale. L'identification de la meilleure correspondance entre le courant ou le potentiel injecté et la distribution de la conductivité peut conduire à une faible résolution spatiale en raison du caractère mal posé du problème.

Il existe donc un besoin pour proposer un procédé de traitement de mesures par TIE qui remédie aux inconvénients de l'art antérieur, notamment pour améliorer la résolution spatiale des images.

Le but de l'invention est de répondre au moins partiellement à ce besoin.

**Exposé de l'invention**

Pour ce faire, l'invention a pour objet, selon un de ses aspects, un procédé de reconstruction de la distribution des propriétés électriques d'un corps comportant une partie cylindrique au sein de laquelle s'écoule un fluide, utilisant un réseau de neurones et des données de valeurs électriques du corps mesurées au préalable par tomographie par impédance électrique à l'aide d'électrodes agencées autour d'une périphérie de la partie cylindrique du corps, chaque électrode ayant été excitée par un potentiel de forme prédéfinie,

le réseau de neurones comportant :

- un auto-encodeur comprenant plusieurs niveaux $L_1,...,L_J$ comportant chacun au moins une couche de convolution et au moins une couche d'abandon, et
- un module d'attention comprenant des portes d'attention $AG_1,..., AG_{J-1}$, ayant chacune un signal d'attention $g_j$ associé,

le procédé comportant les étapes suivantes :

i) les données de valeurs électriques sont traitées successivement par chaque niveau $L_1,...,L_J$ de couches de convolution et de couches d'abandon de l'auto-encodeur du réseau de neurones de sorte à coder les données pour obtenir des données encodées $x_1,..., x_J$ pour chaque niveau de couches $L_1,...,L_J$,

ii) afin de calculer le coefficient $\alpha j$ d'une porte d'attention $AG_j$, cette dernière est configurée pour additionner les données encodées $x_1,..., x_J$ au signal d'attention $g_j$ de la porte d'attention $AG_j$ correspondante, fonction au moins de la concaténation des données de sortie $\hat{x}_{j+1}$ de la porte d'attention de rang supérieur $AG_{j+1}$ et des données décodées provenant du niveau de rang supérieur $L_{j+2}$ à celui de la porte d'attention de rang supérieur $AG_{j+1}$, le résultat de cette addition étant transformé par au moins une fonction mathématique de sorte à obtenir le coefficient $\alpha_j$,

iii) les données de sortie $\hat{x}_j$ d'un niveau de couches $L_j$ de l'auto-encodeur sont multipliées par le coefficient $\alpha_j$ de la porte d'attention correspondante $AG_j$ pour obtenir des données de sortie $\hat{x}_j$ de la première porte d'attention $AG_j$,

iv) l'étape précédente est renouvelée jusqu'à une première porte d'attention $AG_1$ afin d'obtenir les données de sortie $\hat{x}_1$ de la première porte d'attention $AG_1$,

v) lesdites données de sortie $\hat{x}_1$ de la première porte d'attention $AG_1$ sont concaténées avec les données décodées provenant du deuxième niveau $L_2$ afin d'obtenir une matrice de données représentative d'une image de la distribution des propriétés électriques d'au moins un matériau du corps.

Grâce à l'invention, il est possible de reconstruire la distribution des propriétés électriques des matériaux, notamment la conductivité et la permittivité, avec une meilleure résolution spatiale.

Le procédé selon l'invention utilise un réseau neuronal de bout en bout, pouvant être nommé Autoencoder Improved Attention-Net (AIA-Net). Le premier segment est un auto-encodeur utilisé comme architecture d'extraction de caractéristiques de remodelage pour l'entrée de tension, et le second segment est un module d'attention amélioré permettant de résoudre le problème inverse et fournissant l'image de distribution des propriétés électriques des matériaux sous test.

Le potentiel d'excitation des électrodes étant de forme prédéfinie connue, il est possible de reconstruire la conductivité électrique et/ou la permittivité électrique. Grâce aux portes d'attention de l'invention, ayant la connaissance de la correspondance entre les valeurs électriques mesurées et les propriétés électriques des matériaux, la résolution spatiale de l'image en sortie est très satisfaisante.

Auto-encodeur

Les couches d'abandon, ou de « dropout », servent, de façon connue, à activer ou non les neurones. Cette technique permet de réduire l'overfitting lors de l'entraînement du modèle. Certains neurones peuvent être temporairement désactivés dans le réseau, ainsi que toutes les connexions entrantes et sortantes correspondantes. Le choix des neurones à désactiver est avantageusement aléatoire.

Dans un mode de réalisation préféré, un échantillonnage par valeur maximale est mis en œuvre d'un niveau de couches $L_1,...,L_J$ à l'autre afin de compresser les données de valeurs électriques. Ce processus est appelé, de façon connue, « max pooling ». Le procédé de max-pooling s'effectue en passant une fenêtre, ou filtre, de dimension prédéterminée sur l'ensemble de l'entrée. La fenêtre se déplace sur l'image et sélectionne le maximum de chaque portion de l'image, appelée fenêtre ou région de pooling. La fenêtre glisse sur l'entrée et, pour chaque position de la fenêtre, la plus grande valeur est sélectionnée et placée dans la matrice de sortie.

Des connexions de saut sont avantageusement réalisées entre les niveaux de couches $L_1,...,L_J$ pour obtenir les données encodées $x_1,..., x_J$ à l'entrée des portes d'attention. De façon connue, cette technique permet aux réseaux neuronaux convolutifs de contourner certaines couches et de se connecter directement à des couches plus profondes ou moins profondes.

De façon préférée, à l'étape ii), les données encodées $(x_1,.., x_J)$ sont, selon leur dimension spatiale, convoluées ou transposées pour s'adapter à la dimension spatiale du signal d'attention considéré

Module d'attention

Les portes d'attention du module d'attention dans la partie décodage sont avantageusement utilisées comme filtres pour les informations croisées entre la partie codage, en transportant les caractéristiques à travers les connexions de saut, et la partie décodage. Ces informations croisées peuvent être améliorées en ajoutant aux couches supérieures les caractéristiques extraites de chaque couche dans la partie décodage.

Des méthodes connues de tomographie par impédance électrique utilisent l'intelligence artificielle en se concentrant sur la correspondance non linéaire entre la tension et la conductivité, sans donner d'indications au réseau neuronal sur l'endroit où chercher.

Le mécanisme d'attention a été introduit dans l'article [10], où il est formulé comme suit pour l'attention dite douce :

[Math 1]

$$q_{att}^l = \psi^T \left( \sigma_1 \left( W_x^T x_i^l + W_g^T g_i + b_g \right) \right) + b_\psi$$

[Math 2]

$$\alpha_i^l = \sigma_2 (q_{att}^l \left( x_i^l, g_i; \Theta_{att} \right))$$

De façon préférée, le coefficient $\alpha_j$ de la porte d'attention $AG_j$ correspondante est calculé grâce aux équations suivantes :

[Math 3]

$$q_{att}^{l} = \psi^{T}\left(\sigma_1\left(\left(\sum_{j=1}^{4} W_x^T x_{i,j}^l + b_{x_{i,j}}\right) + W_g^T g_{i,j} + b_{g_{i,j}}\right)\right) + b_{\psi}$$

[Math 4]

$$\alpha_{i,j}^{l} = \sigma_2(q_{att}^{l}(x_{i,j}^{l}, g_{i,j}; \Theta_{att}))$$

Où . $q_{att}^{l}$ est le quotient d'attention utilisé pour déterminer les coefficients d'attention $\alpha_j$, $\sigma_1$ et $\sigma_2$ sont respectivement des fonctions d'activation d'unité linéaire rectifiée, notée ReLU, et sigmoïde, $W_x^T$, $W_g^T$ et $\psi^T$ sont des transformations linéaires destinées à réduire la consommation de paramètres et de ressources informatiques, combinées aux termes de biais $b_{\psi}$, $b_{x_{i,j}}$, and $b_{g_{i,j}}$ pour former un ensemble de paramètres $\Theta_{att}$.

Le module d'attention permet de mettre en évidence les régions importantes pour l'extraction des caractéristiques lors de l'exécution des couches de convolution, et de supprimer les régions non pertinentes de l'image.

De préférence, l'opération d'addition des données encodées est effectuée sur toutes les données d'entrée avec chaque signal d'attention $g_j$, principalement afin d'augmenter les poids alignés et diminuer les poids non alignés, ce qui réduit l'information spatiale et met en évidence les caractéristiques importantes.

Les caractéristiques extraites de l'entrée sont avantageusement sélectionnées grâce aux informations contenues dans les signaux d'attention $g_j$.

Les caractéristiques d'entrée $x_1$,..., $x_J$ sont avantageusement mises à l'échelle par rapport aux coefficients d'attention $\alpha_j$, afin d'identifier les zones significatives de l'image, après être passées par une interpolation trilinéaire $W_x^T$, $W_g^T$ et $\psi^T$.

Dans un mode de réalisation préféré, la transformation $\psi$ produit une matrice de poids qui est donnée à la fonction sigmoïde d'activation afin de limiter les valeurs des coefficients d'attention $(\alpha_{i,1}, \alpha_{i,2}, \alpha_{i,3}, \alpha_{i,4}) \varepsilon [0, 1]$.

Un rééchantillonneur peut être utilisé pour étendre les dimensions de la matrice de poids.

À la sortie de chaque porte d'attention, une opération de concaténation peut être effectuée entre le signal d'attention $g_j$ et les données de sortie $\hat{x}$, étant donné qu'ils sont de dimensions différentes.

La multiplication par éléments entre les caractéristiques d'entrée et les coefficients d'attention conduit avantageusement aux données de sortie $(\hat{x}_{i,1}, \hat{x}_{i,2}, \hat{x}_{i,3}, \hat{x}_{i,4})$.

Mesures préalables

Dans un mode de réalisation préféré, la mesure tomographique par impédance électrique du corps, comportant une partie cylindrique contenant un fluide, afin d'obtenir les données de valeurs électriques du corps mesurées au préalable, comporte les étapes suivantes :

i. agencement d'un nombre $n_e$ d'électrodes autour d'une périphérie de la partie cylindrique du corps,
ii. excitation simultanée de chacune des $n_e$ électrodes, chaque électrode étant excitée par un potentiel $V_n^{exc}$ ayant la forme :

[Math 5]

$$V_n^{exc}(t) = A \sum_{m=1}^{n_e} \cos(2\pi f_m t)\left[\delta_m^{\mathbb{O}}\cos(m\theta_n) + \delta_m^{\mathbb{E}}\sin\left(\frac{m\theta_n}{2}\right)\right]$$

où A est une amplitude de signal, $\theta_n$ est la position angulaire de l'électrode $E_n$, $f_m = m * f_0$ est une fréquence d'oscillation, $f_0$ est une fréquence fondamentale choisie telle que $f_m$ soit inférieure à la fréquence de Nyquist du système pour tout m,

iii. mesure des propriétés électriques du corps à l'aide des $n_e$ électrodes,

iv. traitement des données issues de l'étape iii de mesure, comportant les sous-étapes suivantes :

a) pour chaque électrode $E_n$, calcul des points de données $M_n$ définis par :

[Math 7]

$$M_n(k) = \frac{1}{RP}\left|\sum_{p=0}^{P-1} V_n^{meas}(p)e^{ik\beta_p}\right|$$

où R est la valeur de la résistance utilisée pour la mesure de $V_n^{meas}$ avec $V_n^{meas} = R\,I_n$ aux bornes de la résistance, P est le nombre de points d'une séquence discrète de mesure du courant $I_n$, p est le temps discret, k est un coefficient de Fourier compris entre 1 et $(n_e - 1)$ et $\beta_p = (2\pi p/P)$.

Utilisation du procédé

L'invention concerne également l'utilisation du procédé qui vient d'être décrit pour la reconstruction de la distribution des propriétés électriques d'un conduit d'installation nucléaire, un écoulement diphasique traversant notamment le conduit.

Produit programme d'ordinateur

L'invention concerne également, sous un autre de ses aspects, un produit programme d'ordinateur comportant un support et, enregistrées sur ce support, des instructions lisibles par un processeur pour que, lorsqu'exécutées, elles permettent de mettre en œuvre le procédé de reconstruction selon l'invention.

Dispositif

L'invention concerne enfin un dispositif pour la mise en œuvre du procédé selon l'invention, comportant :

- un système d'acquisition comportant au moins un réseau logique programmable, un module de génération de signaux analogiques et un module de mesure de signaux analogiques, une pluralité d'électrodes étant notamment connectées au système d'acquisition,
- un ordinateur configuré pour contrôler le système d'acquisition,
- un réseau de neurones intégré ou relié à l'ordinateur, le réseau de neurones comportant :

  - un auto-encodeur comprenant plusieurs niveaux $L_1,...,L_J$ comportant chacun au moins une couche de convolution et au moins une couche d'abandon, et
  - un module d'attention comprenant des portes d'attention $AG_1,..., AG_{J-1}$, ayant chacune un signal d'attention $g_j$ associé.

Les caractéristiques énoncées ci-dessus pour le procédé s'appliquent à l'utilisation, au produit programme d'ordinateur et au dispositif, et vice versa.

**Brève description des dessins**

[Fig 1] La figure 1 représente un diagramme illustrant un exemple d'étapes de mise en œuvre du procédé selon l'invention,

[Fig 2] La figure 2 illustre un exemple d'architecture pour la mise en œuvre du procédé selon l'invention.

[Fig 3a] [Fig 3b) Les figures 3a et 3b représentent en détails un exemple de portes d'attention utilisées dans l'invention,

[Fig 4] La figure 4 illustre des résultats comparatifs entre le procédé selon l'invention et l'art antérieur, et

[Fig 5] La figure 5 représente une courbe illustrant l'erreur quadratique moyenne obtenue lors de l'utilisation du procédé selon l'invention.

## Description détaillée d'un exemple

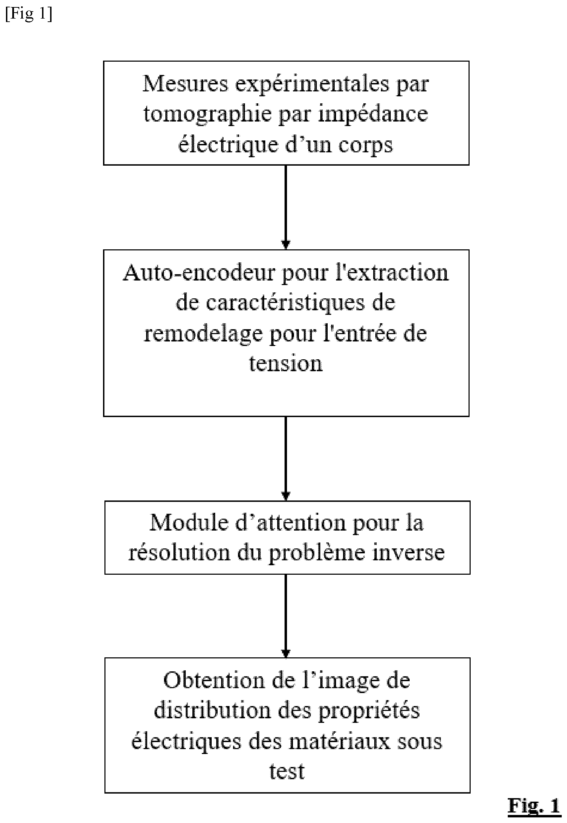

On a illustré en figure 1 un diagramme représentant les étapes d'un exemple de mise en œuvre du procédé selon l'invention.

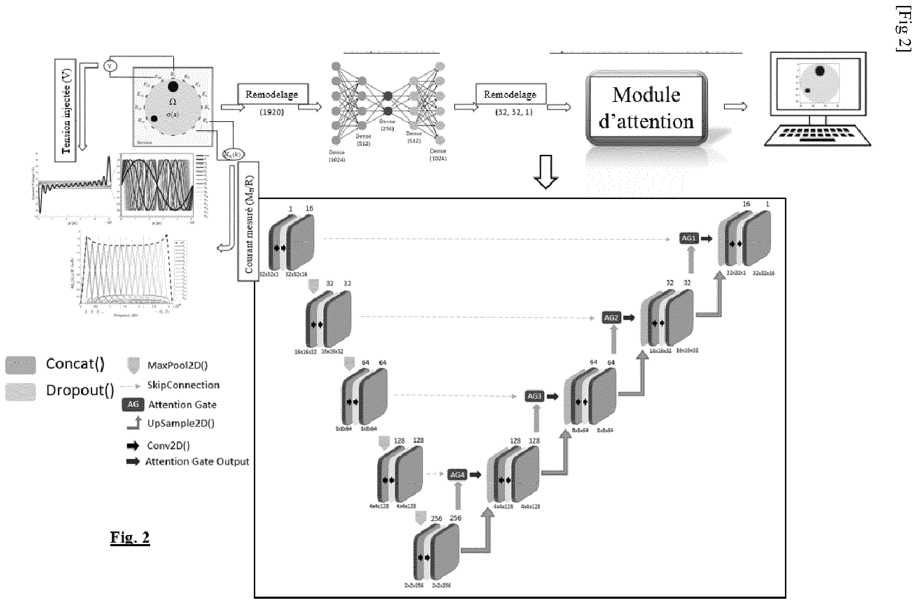

Lors d'une première étape, des mesures expérimentales par tomographie par impédance électrique sont réalisées sur un corps comportant une partie cylindrique à l'intérieur de laquelle s'écoule un fluide, à l'aide d'électrodes agencées autour d'une périphérie de la partie cylindrique du corps, chaque électrode ayant été excitée par un potentiel de forme prédéfinie. Les électrodes sont disposées de façon non-intrusive sur une périphérie circulaire du corps, comme visible à la figure 2. Les électrodes sont dans cet exemple angulairement réparties de manière régulière autour de la périphérie du corps.

Les électrodes sont reliées à un circuit imprimé, lui-même connecté à un système d'acquisition de données. Un écran permet de visualiser les données et les images produites à partir de ces données. Le système d'acquisition de données contient le système d'exploitation linux (HÔTE) qui contrôle un réseau logique programmable FPGA, également contenu dans le système d'acquisition de données.

Le système d'acquisition permet de générer les signaux analogiques d'excitation et de mesurer les signaux analogiques de mesure en provenance des électrodes.

Des données de valeurs électriques $M_n(k)$ sont ainsi obtenues.

Dans une deuxième étape, ces données sont traitées par l'auto-encodeur du réseau de neurones selon l'invention afin de les remodeler pour obtenir des données encodées $x_1,..., x_J$ pour chaque niveau de couches $L_1,...,L_J$ de l'auto-encodeur, comme visible à la figure 2. Chaque niveau comporte au moins une couche de convolution et au moins une couche d'abandon, dite de « dropout ». Les données sont traitées par couches et concaténées entre elles.

Comme visible à la figure 2, un échantillonnage par valeur maximale, dit « max-pooling », est mis en œuvre d'un niveau de couches $L_1,...,L_J$ à l'autre afin de compresser les données de valeurs électriques.

Dans une troisième étape, le module d'attention selon l'invention est utilisé pour la résolution du problème inverse. Ce module d'attention comprend des portes d'attention $AG_1,..., AG_{J-1}$, ayant chacune un signal d'attention $g_j$ associé.

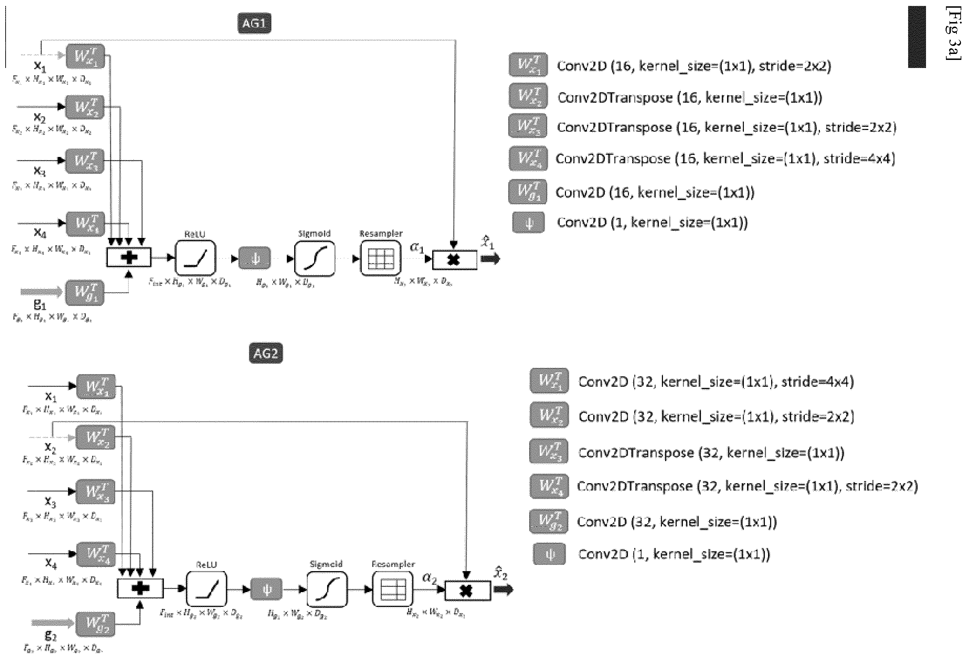

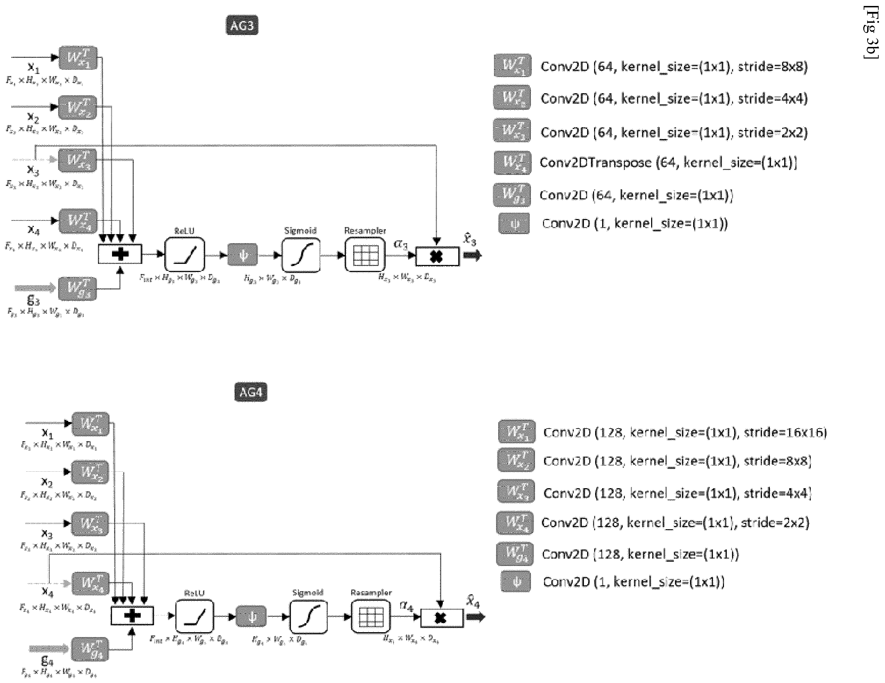

Comme représenté aux figures 3a et 3b, afin de calculer le coefficient $\alpha j$ d'une porte d'attention $AG_j$, cette dernière est configurée pour additionner les données encodées $x_1,..., x_J$ au signal d'attention $g_j$ de la porte d'attention $AG_j$ correspondante, fonction au moins de la concaténation des données de sortie $\hat{x}_{j+1}$ de la porte d'attention de rang supérieur $AG_{j+1}$ et des données décodées provenant du niveau de rang supérieur $L_{j+2}$ à celui de la porte d'attention de rang supérieur $AG_{j+1}$. Le résultat de cette addition est transformé par au moins une fonction mathématique de sorte à obtenir le coefficient $\alpha_j$.

De préférence et dans l'exemple considéré, le coefficient $\alpha_j$ de la porte d'attention $AG_j$ correspondante est calculé grâce aux équations suivantes :

[Math 3]

$$q_{att}^l = \psi^T\left(\sigma_1\left(\left(\sum_{j=1}^{4} W_x^T x_{i,j}^l + b_{x_{i,j}}\right) + W_g^T g_{i,j} + b_{g_{i,j}}\right)\right) + b_\psi$$

[Math 4]

$$\alpha_{i,j}^l = \sigma_2\left(q_{att}^l\left(x_{i,j}^l, g_{i,j}; \Theta_{att}\right)\right)$$

où $q_{att}^l$ est le quotient d'attention utilisé pour déterminer les coefficients d'attention $\alpha j$, $\sigma_1$ et $\sigma_2$ sont respectivement des fonctions d'activation d'unité linéaire rectifiée, notée ReLU, et sigmoïde, $W_x^T$, $W_g^T$ et $\psi^T$ sont des transformations linéaires destinées à réduire la consommation de paramètres et de ressources informatiques, combinées aux termes de

biais $b_\psi$, $b_{x_{i,j}}$, and $b_{g_{i,j}}$ pour former un ensemble de paramètres $\Theta_{att}$.

La transformation $\psi$ produit une matrice de poids qui est donnée à la fonction sigmoïde d'activation afin de limiter les valeurs des coefficients d'attention, un rééchantillonneur est utilisé pour étendre les dimensions de la matrice de poids.

Dans l'exemple illustré, comme visible à la figure 2, des connexions de saut sont réalisées entre les niveaux de couches $L_1,...,L_J$ pour obtenir les données encodées $x_1,..., x_J$ à l'entrée des portes d'attention.

Lors de l'étape du calcul des coefficients d'attention, les données encodées $x_1,..., x_J$ sont, selon leur dimension spatiale, convoluées ou transposées pour s'adapter à la dimension spatiale du signal d'attention considéré, comme représenté aux figures 3a et 3b.

Par exemple, dans la porte d'attention $AG_1$, la dimension spatiale de $x_1$ est supérieure, du double, à celle du signal d'attention $g_1$. Pour pouvoir les additionner, il convient donc de diviser la dimension de $x_1$ par 2 (Conv2D avec stride = 2). En ce qui concerne les autres signaux $x_j$ en entrée, leur dimension spatiale est inférieure à celle du signal d'attention $g_1$, sauf pour $x_2$ qui est au même niveau de dimension. Il convient ainsi d'effectuer une convolution inverse avec des pas différents (stride = 1 pour $x_2$, 2 pour $x_3$ et 4 pour $x_4$) afin d'additionner les signaux avec $g_1$.

Le même principe se poursuit pour $AG_2$, $AG_3$, $AG_4$.

Sur cette figure, F, H, W, D, $F_{int}$, sont respectivement les caractéristiques, la hauteur, la largeur, le canal d'entrée ou la profondeur, dans le cas de données 3D, et les caractéristiques intermédiaires.

Les données de sortie $x_J$ d'un niveau de couches $L_j$ de l'auto-encodeur sont multipliées par le coefficient $\alpha_j$ de la porte d'attention correspondante $AG_j$, ainsi calculé, afin d'obtenir des données de sortie $\hat{x}_j$ de la première porte d'attention $AG_j$, tel qu'illustré aux figures 3a et 3b. Cette étape est renouvelée jusqu'à une première porte d'attention $AG_1$ afin d'obtenir les données de sortie $\hat{x}_1$ de la première porte d'attention $AG_1$.

Comme visible à la figure 2, les données de sortie $\hat{x}_1$ de la première porte d'attention ($AG_1$) sont concaténées avec les données décodées provenant du deuxième niveau ($L_2$) afin d'obtenir, dans la dernière étape de la figure 1, une matrice de données représentative d'une image de la distribution des propriétés électriques d'au moins un matériau du corps. Dans cet exemple, l'image est une matrice 32 x 32.

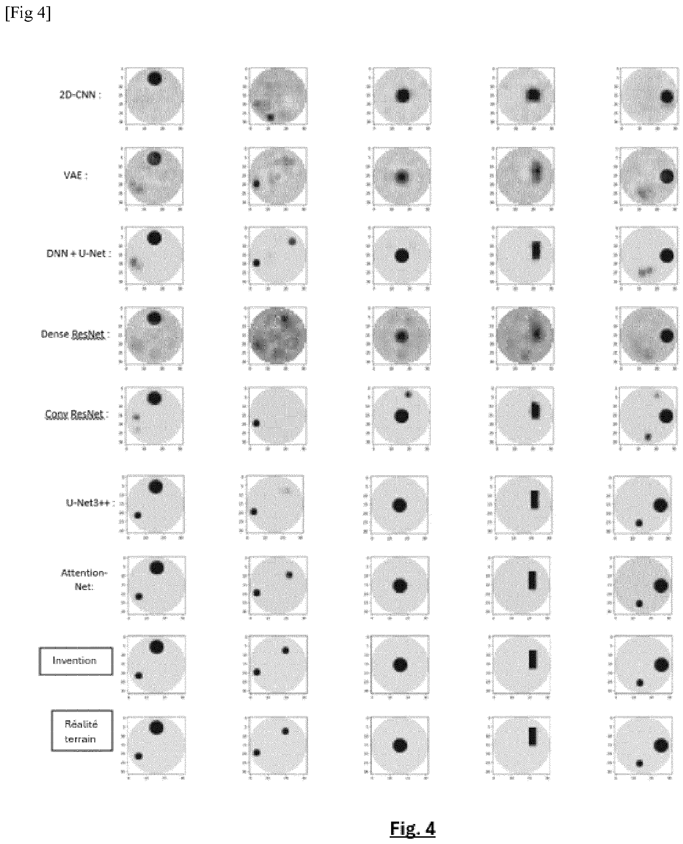

La figure 4 présente une partie des résultats obtenus grâce à la mise en œuvre du procédé selon l'invention et comparés à d'autres architectures connues : 2D-CNN, Variational-AutoEncoder (VAE), Generative DNN with U-Net, Dense ResNet, Conv ResNet, U-Net3++, Attention-Net.

Les images de gauche à droite sur la figure 4 correspondent à des tests réalisés sur un corps ayant une partie cylindrique qui peut comporter :

- deux bâtons de section circulaire (de 1 cm et 2 cm de diamètre),

- deux bâtons de section circulaire (de 1 cm de diamètre),

- un bâton de section circulaire (de 2 cm de diamètre),

- un bâton de section rectangulaire (de 2.5 $\times$ 1 cm de surface), et

- deux bâtons de section circulaire (de 1 cm et 2 cm de diamètre).

Il est à noter que, dans un souci de concision, seuls les résultats de certains tests ont été illustrés mais que les conclusions sont les mêmes pour tous les tests réalisés.

Nous pouvons constater qu'avant U-Net3++, les architectures connues n'étaient pas suffisamment optimisées pour reconstruire la conductivité, principalement en raison du problème du gradient de fuite, puisqu'il n'y a pas de connexions sautées. L'exception serait l'architecture DNN+U-Net dans laquelle U-Net inclut ces connexions sautées mais l'extraction des caractéristiques est initiée avec un réseau de neurones profond, qui n'est pas optimisé pour les données d'image par rapport à un réseau de neurones convolutif. Pour U-Net3++, en regardant la deuxième image de gauche à droite, le réseau manquait d'identification des caractéristiques spatiales pour une reconstruction précise, ce qui a été amélioré avec le réseau classique Attention-Net, mais ce réseau de neurones a eu des problèmes avec la conductivité de l'eau en arrière-plan et peut être vu dans l'image N°5 de la figure 4. Le réseau utilisé dans l'invention montre de très bons résultats de reconstruction avec une meilleure identification des bords spatiaux.

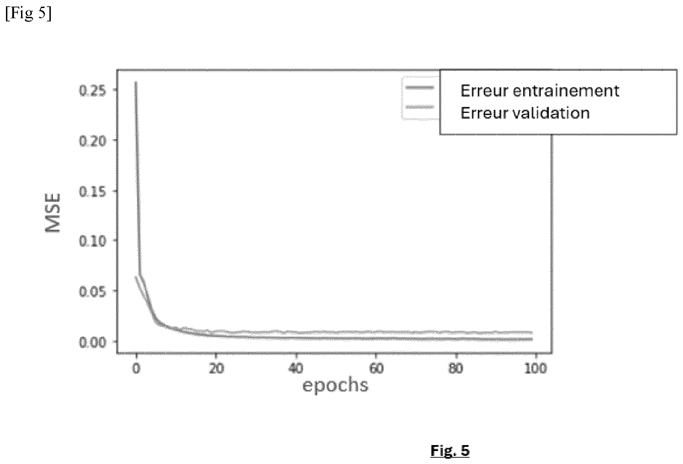

Le tableau 1 montre que les mesures donnent de meilleurs résultats avec l'invention. Même si le $R^2$ a une valeur élevée pour l'architecture proposée, il peut conduire à de mauvaises interprétations. Les valeurs de RMSE et SER sont assez faibles, ce qui est le signe d'une bonne régression, et se situent dans la plage de la fonction de perte MSE, comme illustré à la figure 5.

[Table 1]

| Architectures | Métriques | | |
|---|---|---|---|
| | $R^2$ (%) | RMSE ($\mu$S/cm) | SER ($\mu$S/cm) |
| 2D-CNN | 68.3 | 0.3001 | 0.3240 |
| VAE | 70.9 | 0.2769 | 0.2831 |
| DNN + U-Net | 76.3 | 0.2547 | 0.2342 |
| Dense ResNet | 80.4 | 0.2010 | 0.1912 |
| Conv ResNet | 85.9 | 0.1832 | 0.1447 |
| U-Net3++ | 91.7 | 0.1521 | 0.1272 |
| Attention-Net | 92.3 | 0.1308 | 0.1187 |
| **AIA-Net** | **98.5** | **0.1012** | **0.1009** |

La figure 5 montre que la fonction de perte de l'erreur quadratique moyenne est très faible, ce qui indique une bonne correspondance entre les données d'entrée de tension et les données de sortie de conductivité. La perte de validation présente de faibles variations, ce qui indique que le modèle du réseau de neurones mis en œuvre est stable. En outre, l'absence de surajustement ou de sous-ajustement est expliquée par les valeurs proches entre les deux pertes.

L'invention n'est pas limitée aux exemples qui viennent d'être décrits ; on peut notamment combiner entre elles des caractéristiques des exemples illustrés au sein de variantes non illustrées.

D'autres variantes et améliorations peuvent être envisagées sans pour autant sortir du cadre de l'invention. En particulier, le procédé selon l'invention peut être mis en œuvre au moyen d'un réseau de neurones différent de celui décrit, comportant notamment des couches supplémentaires.

L'invention peut être utilisée dans le domaine nucléaire, notamment comme méthode de prévention lors de présence de bulles dans le circuit primaire, et de cavitation dans les pompes des différents circuits. L'invention peut être appliquée au domaine médical, en particulier pour la supervision des poumons et la détection d'anomalies, en mettant notamment en œuvre une excitation simultanée.

Le procédé selon l'invention peut trouver une application dans le domaine agroalimentaire, notamment pour le comptage de fruits et légumes, dans le domaine pétrolier, en particulier pour la détection d'encrassement dans les puits de production, ou encore dans le domaine pharmaceutique, par exemple pour la détection de corps étrangers lors de l'usinage de médicaments.

**Liste des références cités**

[1] R. Guardo & al., "A Neural Network Approach To Image Reconstruction In Electrical Impedance Tomography", IEEE, 1991

[2] A. Nejatali & al., "An iterative algorithm for electrical impedance imaging using neural networks", IEEE, 1998

[3] J. Lampinen & al., "Application of Bayesian neural network in electrical impedance tomography", IEEE, 1999

[4] Chao Wang & al., "RBF neural network image reconstruction for electrical impedance tomography", IEEE, 2004

[5] Xiuyan Li & al., "An image reconstruction framework based on deep neural network for electrical impedance tomography", IEEE, 2017

[6] Jin K. S. & al., "A Learning-Based Method for Solving Ill-Posed Nonlinear Inverse Problems: ASimulation Study of Lung EIT", SIAM J. Imaging sciences, 2019

[7] Xiaoyan C. & al., "Deep Autoencoder Imaging Method for Electrical Impedance Tomography", IEEE, 2021

[8] Akarsh P. & al., "Improved Training of Physics-Informed Neural Networks Using Energy-Based Priors: a Study on Electrical Impedance Tomography", ICLR, 2023

[9] Hao Y. & al., "High-resolution conductivity reconstruction by electrical impedance tomography using structure-aware hybrid-fusion learning", Sciencedirect, 2024

[10] Ozan Oktay & al., "AttentionU-Net : Learning Where to look for the Pancreas," 1st Conference on MIDL, 2018

**Revendications**

**1.** Procédé de reconstruction de la distribution des propriétés électriques d'un corps comportant une partie cylindrique au sein de laquelle s'écoule un fluide, utilisant un réseau de neurones et des données de valeurs électriques du corps mesurées au préalable par tomographie par impédance électrique à l'aide d'électrodes agencées autour d'une périphérie de la partie cylindrique du corps, chaque électrode ayant été excitée par un potentiel de forme prédéfinie,

le réseau de neurones comportant :

- un auto-encodeur comprenant plusieurs niveaux ($L_1$,...,$L_J$) comportant chacun au moins une couche de convolution et au moins une couche d'abandon, et
- un module d'attention comprenant des portes d'attention ($AG_1$,..., $AG_{J-1}$), ayant chacune un signal d'attention ($g_j$) associé,

le procédé comportant les étapes suivantes :

i) les données de valeurs électriques sont traitées successivement par chaque niveau ($L_1$,...,$L_J$) de couches de convolution et de couches d'abandon de l'auto-encodeur du réseau de neurones de sorte à coder les données pour obtenir des données encodées ($x_1$,.., $x_J$) pour chaque niveau de couches ($L_1$,...,$L_J$),

ii) afin de calculer le coefficient ($\alpha_j$) d'une porte d'attention ($AG_j$), cette dernière est configurée pour additionner les données encodées ($x_1$,.., $x_J$) au signal d'attention ($g_j$) de la porte d'attention ($AG_j$) corres-pondante, fonction au moins de la concaténation des données de sortie $\widehat{(x_{j+1})}$ de la porte d'attention de rang supérieur ($AG_{j+1}$) et des données décodées provenant du niveau de rang supérieur ($L_{j+2}$) à celui de la porte d'attention de rang supérieur ($AG_{j+1}$), le résultat de cette addition étant transformé par au moins une fonction mathématique de sorte à obtenir le coefficient ($\alpha_j$),

iii) les données de sortie ($x_J$) d'un niveau de couches ($L_J$) de l'auto-encodeur sont multipliées par le coefficient ($\alpha_j$) de la porte d'attention correspondante ($AG_j$) pour obtenir des données de sortie $\widehat{(x_j)}$ de la première porte d'attention ($AG_j$),

iv) l'étape précédente est renouvelée jusqu'à une première porte d'attention ($AG_1$) afin d'obtenir les données de sortie ($\hat{x}_1$) de la première porte d'attention ($AG_1$),

v) lesdites données de sortie ($\hat{x}_1$) de la première porte d'attention ($AG_1$) sont concaténées avec les données décodées provenant du deuxième niveau ($L_2$) afin d'obtenir une matrice de données représentative d'une image de la distribution des propriétés électriques d'au moins un matériau du corps.

**2.** Procédé selon la revendication 1, dans lequel un échantillonnage par valeur maximale est mis en œuvre d'un niveau de couches ($L_1$,...,$L_J$) à l'autre afin de compresser les données de valeurs électriques.

**3.** Procédé selon l'une quelconque des revendications précédentes, dans lequel des connexions de saut sont réalisées entre les niveaux de couches ($L_1$,...,$L_J$) pour obtenir les données encodées ($x_1$,.., $x_J$) à l'entrée des portes d'attention.

**4.** Procédé selon l'une quelconque des revendications précédentes, dans lequel, à l'étape ii), les données encodées ($x_1$,.., $x_J$) sont, selon leur dimension spatiale, convoluées ou transposées pour s'adapter à la dimension spatiale du signal d'attention considéré.

**5.** Procédé selon l'une quelconque des revendications précédentes, dans lequel le coefficient ($\alpha_j$) de la porte d'attention ($AG_j$) correspondante est calculé grâce aux équations suivantes :

[Math 3]

$$q_{att}^l = \psi^T \left( \sigma_1 \left( \left( \left( \sum_{j=1}^{4} W_x^T x_{i,j}^l + b_{x_{i,j}} \right) + W_g^T g_{i,j} + b_{g_{i,j}} \right) \right) \right) + b_\psi$$

[Math 4]

$$\alpha_{i,j}^l = \sigma_2 \left( q_{att}^l \left( x_{i,j}^l, g_{i,j}; \Theta_{att} \right) \right)$$

où $q_{att}^l$ est le quotient d'attention utilisé pour déterminer les coefficients d'attention $\alpha j$, $\sigma_1$ et $\sigma_2$ sont respectivement des fonctions d'activation d'unité linéaire rectifiée, notée ReLU, et sigmoïde, $W_x^T$, $W_g^T$ et $\psi^T$ sont des transformations linéaires destinées à réduire la consommation de paramètres et de ressources informatiques, combinées aux termes de biais $b_\psi$, $b_{x_{i,j}}$, and $b_{g_{i,j}}$ pour former un ensemble de paramètres $\Theta_{att}$.

6. Procédé selon la revendication précédente, dans lequel, la transformation ($\psi$) produisant une matrice de poids qui est donnée à la fonction sigmoïde d'activation afin de limiter les valeurs des coefficients d'attention, un rééchantillonneur est utilisé pour étendre les dimensions de la matrice de poids.

7. Utilisation du procédé selon l'une des revendications précédentes pour la reconstruction de la distribution des propriétés électriques d'un conduit d'installation nucléaire, un écoulement diphasique traversant notamment le conduit.

8. Produit programme d'ordinateur comportant un support et, enregistrées sur ce support, des instructions lisibles par un processeur pour que, lorsqu'exécutées, elles permettent de mettre en œuvre le procédé de reconstruction selon l'une quelconque des revendications 1 à 6.

9. Dispositif pour la mise en œuvre du procédé selon l'une quelconque des revendications 1 à 6, comportant :

   - un système d'acquisition comportant au moins un réseau logique programmable, un module de génération de signaux analogiques et un module de mesure de signaux analogiques, une pluralité d'électrodes étant notamment connectées au système d'acquisition,
   - un ordinateur configuré pour contrôler le système d'acquisition,
   - un réseau de neurones intégré ou relié à l'ordinateur, le réseau de neurones comportant :

      - un auto-encodeur comprenant plusieurs niveaux ($L_1$,...,$L_J$) comportant chacun au moins une couche de convolution et au moins une couche d'abandon, et
      - un module d'attention comprenant des portes d'attention ($AG_1$,..., $AG_{J-1}$), ayant chacune un signal d'attention ($g_j$) associé.

[Fig 1]

```
┌─────────────────────────────────┐
│   Mesures expérimentales par    │
│   tomographie par impédance     │
│     électrique d'un corps       │
└─────────────────────────────────┘
                │
                ▼
┌─────────────────────────────────┐
│  Auto-encodeur pour l'extraction│
│      de caractéristiques de     │
│   remodelage pour l'entrée de   │
│             tension             │
└─────────────────────────────────┘
                │
                ▼
┌─────────────────────────────────┐
│    Module d'attention pour la   │
│  résolution du problème inverse │
└─────────────────────────────────┘
                │
                ▼
┌─────────────────────────────────┐
│    Obtention de l'image de      │
│  distribution des propriétés    │
│ électriques des matériaux sous  │
│             test                │
└─────────────────────────────────┘
```

**Fig. 1**

[Fig 2]

Fig. 2

[Fig 3a]

AG1

$x_1$

$W_{x_1}^T$

$x_2$

$W_{x_2}^T$

$x_3$

$W_{x_3}^T$

$x_4$

$W_{x_4}^T$

$g_1$

$W_{g_1}^T$

ReLU

$\Psi$

Sigmoid

Resampler

$\alpha_1$

$\hat{x}_1$

$W_{x_1}^T$   Conv2D (16, kernel_size=(1x1), stride=2x2)

$W_{x_2}^T$   Conv2DTranspose (16, kernel_size=(1x1))

$W_{x_3}^T$   Conv2DTranspose (16, kernel_size=(1x1), stride=2x2)

$W_{x_4}^T$   Conv2DTranspose (16, kernel_size=(1x1), stride=4x4)

$W_{g_1}^T$   Conv2D (16, kernel_size=(1x1))

$\Psi$   Conv2D (1, kernel_size=(1x1))

AG2

$x_1$

$W_{x_1}^T$

$x_2$

$W_{x_2}^T$

$x_3$

$W_{x_3}^T$

$x_4$

$W_{x_4}^T$

$g_2$

$W_{g_2}^T$

ReLU

$\Psi$

Sigmoid

Resampler

$\alpha_2$

$\hat{x}_2$

$W_{x_1}^T$   Conv2D (32, kernel_size=(1x1), stride=4x4)

$W_{x_2}^T$   Conv2D (32, kernel_size=(1x1), stride=2x2)

$W_{x_3}^T$   Conv2DTranspose (32, kernel_size=(1x1))

$W_{x_4}^T$   Conv2DTranspose (32, kernel_size=(1x1), stride=2x2)

$W_{g_2}^T$   Conv2D (32, kernel_size=(1x1))

$\Psi$   Conv2D (1, kernel_size=(1x1))

14

[Fig 3b]

[Fig 4]

**Fig. 4**

[Fig 5]

**Fig. 5**

Europäisches
Patentamt

European
Patent Office

Office européen
des brevets

# RAPPORT DE RECHERCHE EUROPEENNE

Numéro de la demande

EP 25 17 4281

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | YU HAO ET AL: "Multiscale Voltage Reconstruction With Attention-Based Network for Volume Fraction Prediction of Industrial Oil-Water Two-Phase Flow by EIT", IEEE TRANSACTIONS ON INSTRUMENTATION AND MEASUREMENT, vol. 71, 1 janvier 2022 (2022-01-01), pages 1-9, XP093239683, USA ISSN: 0018-9456, DOI: 10.1109/TIM.2022.3169557 * le document en entier * | 1-9 | INV. A61B5/0536 G06N3/0464 |
| A | XINXIN GAO ET AL: "A hybrid deep learning model for ECT image reconstruction of cryogenic fluids", FLOW MEASUREMENT AND INSTRUMENTATION., vol. 87, 1 octobre 2022 (2022-10-01), page 102228, XP093239932, GB ISSN: 0955-5986, DOI: 10.1016/j.flowmeasinst.2022.102228 * figures 1-4 * | 1-9 | |
| A | DONG QINGHE ET AL: "Image reconstruction method for electrical impedance tomography based on RBF and attention mechanism", COMPUTERS & ELECTRICAL ENGINEERING, PERGAMON PRESS, GB, vol. 110, 3 juillet 2023 (2023-07-03), XP087383946, ISSN: 0045-7906, DOI: 10.1016/J.COMPELECENG.2023.108826 [extrait le 2023-07-03] * le document en entier * | 1-9 | |

-----

-----

-----

-/--

DOMAINES TECHNIQUES RECHERCHES (IPC)

A61B
G06N

Le présent rapport a été établi pour toutes les revendications

| Lieu de la recherche | Date d'achèvement de la recherche | Examinateur |
|---|---|---|
| Berlin | 5 septembre 2025 | Kronberger, Raphael |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...........................................................................

& : membre de la même famille, document correspondant

EPO FORM 1503 03.82 (P04C02)

EP 4 647 004 A1

**RAPPORT DE RECHERCHE EUROPEENNE**

Numéro de la demande

EP 25 17 4281

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | ZHANG BAOJIE ET AL: "HybridDenseU-Net: learning a multi-scale convolution and dense connectivity CNN for inverse imaging problems", MEASUREMENT SCIENCE AND TECHNOLOGY, IOP, BRISTOL, GB, vol. 35, no. 3, 11 décembre 2023 (2023-12-11), XP020486253, ISSN: 0957-0233, DOI: 10.1088/1361-6501/AD11CD [extrait le 2023-12-11] * le document en entier * ----- | 1-9 | |
| A | CN 117 911 713 A (UNIV TIANJIN POLYTECHNIC) 19 avril 2024 (2024-04-19) * le document en entier * ----- | 1-9 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 5 septembre 2025 | Kronberger, Raphael |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 25 17 4281

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

05-09-2025

| Document brevet cité au rapport de recherche | Date de publication | Membre(s) de la famille de brevet(s) | Date de publication |
|---|---|---|---|
| CN 117911713 A | 19-04-2024 | AUCUN | |

Pour tout renseignement concernant cette annexe : voir Journal Officiel de l'Office européen des brevets, No.12/82

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3121234

- WO 202277866 A

**Littérature non-brevet citée dans la description**

- **R. GUARDO**. A Neural Network Approach To Image Reconstruction In Electrical Impedance Tomography. IEEE, 1991
- **A. NEJATALI**. An iterative algorithm for electrical impedance imaging using neural networks. IEEE, 1998
- **J. LAMPINEN**. Application of Bayesian neural network in electrical impedance tomography. IEEE, 1999
- **CHAO WANG**. RBF neural network image reconstruction for electrical impedance tomography. IEEE, 2004
- **XIUYAN LI**. An image reconstruction framework based on deep neural network for electrical impedance tomography. IEEE, 2017

- **JIN K. S.** A Learning-Based Method for Solving Ill-Posed Nonlinear Inverse Problems: ASimulation Study of Lung EIT. *SIAM J. Imaging sciences*, 2019
- **XIAOYAN C.** Deep Autoencoder Imaging Method for Electrical Impedance Tomography. IEEE, 2021
- **AKARSH P.** Improved Training of Physics-Informed Neural Networks Using Energy-Based Priors: a Study on Electrical Impedance Tomography. *ICLR*, 2023
- **HAO Y.** High-resolution conductivity reconstruction by electrical impedance tomography using structure-aware hybrid-fusion learning. *Sciencedirect*, 2024
- **OZAN OKTAY**. AttentionU-Net : Learning Where to look for the Pancreas. *1st Conference on MIDL*, 2018